# EUROPEAN PATENT APPLICATION

(11) **EP 4 417 137 A1**
(43) Date of publication of application: **21.08.2024**
(21) Application number: 24157695.8
(22) Date of filing: 14.02.2024
(51) Int. Cl.: A61B 10/00, G01N 33/543, G01N 33/94, G01N 21/78, G01N 21/84, G01N 33/487, G01N 33/569, G01N 33/574

(54) **DEVICE WITH MULTIPLE TEST UNITS**

(30) Priority: 15.02.2023 US 202363445814 P
(71) Applicant: Zhejiang Orient Gene Biotech Co., Ltd, Anji Huzhou Zhejiang 313300 (CN)
(72) Inventor: Shen, Lili, Huzhou, 313300 (CN); Fang, Jianqiu, Huzhou, 313300 (CN); Li, Gang, Huzhou, 313300 (CN); Zhou, Fangli, Huzhou, 313300 (CN)
(74) Representative: Piotrowicz, Pawel Jan Andrzej

(57) **Abstract**

A device for detecting analytes in a sample includes a main body (700; 900; 923). The main body (700; 900; 923) includes a plurality of testing units (800; 911, 912, 913, 914, 915, 999). Each testing unit (800; 911, 912, 913, 914, 915, 999) is configured to detect one or more analytes in the sample. Each testing unit (800; 911, 912, 913, 914, 915, 999) is separable from the main body (700; 900; 923), and/or the testing units (800; 911, 912, 913, 914, 915, 999) are connected together through the main body (700; 900; 923). When testing needs to be performed, the testing units (800; 911, 912, 913, 914, 915, 999) are separable from each other, or the testing units (800; 911, 912, 913, 914, 915, 999) are separable from the main body (700; 900; 923). The testing units (800; 911, 912, 913, 914, 915, 999) are inseparable from the main body (700; 923) after completing the testing and entering the main body (700; 923) again.

## Description

### CROSS-REFERENCE TO RELATED APPLICATIONS

The present application claims the priority of US application No. 63/445,814 filed on February 15, 2023, the entire contents of which, including but not limited to specifications, abstracts, accompanying drawings, and claims, are a part of the present application.

### BACKGROUND OF THE INVENTION

### Field of the Invention

The present invention relates to a device with a plurality of testing units, in particular to a device for testing analytes in a liquid sample in the field of rapid diagnosis, such as a urine and saliva collection and test device.

### Description of the Related Art

The following description is merely an introduction of some background knowledge and does not constitute any limitation to the present invention.

At present, the test device for detecting the presence or absence of an analyte in sample is widely used in hospitals or homes, and such test devices for rapid diagnosis include one or more test strips, for example, for early pregnancy testing and drug of abuse testing. Such test devices for rapid diagnosis are very convenient, and can obtain a test result from the test strips in one minute or at most ten minutes or so.

In some cases, samples need to be collected and then tested by professional testing agencies or test laboratories. Some tests need to be completed in the site in time, for example, roadsides, for example, persons who drive after drug use need to be tested on the spot (referred to as "Drug Driving"), to obtain the test results in time. For example, the test of saliva samples is gradually accepted and favored by testing agencies or testing personnel due to convenient collection. In some literatures, various specimen collection and test devices for clinical and domestic uses have been described. For example, the US Patent No. 5,376,337 discloses a saliva sampling device in which a piece of filter paper is used for collecting saliva from the mouth of a subject and delivering the saliva to an indicator reagent. US patents US 5,576,009 and US 5,352,410 each disclose a syringe-type liquid sampling device.

In addition, with the spread of infectious diseases in recent years, especially the spread of coronavirus, home self-testing products have become mainstream products, which are convenient for sampling and friendly to operate for self-testing at home and prevent sampling from polluting the environment and causing reinfection. This puts forward higher requirements for home self-testing products.

Among many in-vitro tests, in small tests or home tests, in order to make the test results more accurate, it is often necessary to use a machine to read the test results. Such test results read by a machine are transmitted as electrical signals or displayed on a display screen as digitized test results, which is convenient for storage and transmission of the test results. The test results are transmitted to a medical center, for example, by wireless transmission. Such test results are all completed by testing units, such as test cards. When a machine needs to test a variety of analytes, it is necessary to prepare a lot of different testing units. These testing units may have the same external structure or appearance, but may each be used for detecting different analytes. For a nonprofessional person, it is difficult to distinguish the analyte of each test card. Even for a professional organization, it is still difficult to distinguish the specific analyte corresponding to each test card. In addition, because of different purposes of testing and different substances to be tested for each test, people which perform testing are often confused, and thus, are prone to incorrect testing.

In view of the technical problems in some traditional products above, it is necessary to improve them and provide another way to solve the defects in existing traditional technologies, so as to meet the increasing demand for in-vitro diagnosis, especially the demand of the home self-testing market.

### BRIEF SUMMARY OF THE INVENTION

In view of the above, in order to overcome the defects in the prior art, the present invention provides a device with multiple testing units. These testing units are detachably connected together at the beginning. After being separately detached to read test results, in some cases, the testing units can be combined together. After being combined again, the testing units are inseparable, which is convenient for disposal. Of course, after the plurality of testing units are separated and used for testing, they cannot be combined together again. Here, the "detachable" means that the testing units are connected detachably or arranged detachably through the same main body where the testing units are located (the testing units can each leave the main body at random), and the "inseparable" means that once combined again after the end of the testing, the testing units cannot be separated from each other, and or cannot be separated from the main body by being combined with the main body. This is convenient for testing, processing or other purposes.

Therefore, in one aspect, the present invention provides a device for detecting analytes in a sample, including a main body. The main body includes a plurality of testing units. Each testing unit is configured to detect one or more analytes. Each testing unit is separable from the main body, or/and the testing units are connected together through the main body. When testing needs to be performed, the testing units are separable from each other, or the testing units are separable from the main body. The testing units are inseparable from the main body after completing the testing and being inserted into the main body again. In this way, the plurality of testing units are combined with the main body, so that the tester can directly remove the testing unit from the main body for testing, and place the testing unit back to the main body after the testing is completed. After being placed back to the main body, the testing unit is inseparable from the main body, which avoids testing a second time. Generally, the testing element can be used for testing once, and cannot be used for testing a second time. This avoids confusion. Particularly, when the test results of the testing units are read by a machine, this prevents the testing units from being inserted into the machine a second time for reading a second time. This is because the test results of the testing units are valid only within a certain period of time, for example, within 5 to 30 minutes, and the test results read at a time less than or more than this time are invalid. However, it is difficult for a machine to judge the time of testing or whether the result has been read or not. If the test card whose test result has been read by the machine is placed back to the main body and made inseparable from the main body, then the card that has been used for testing is unable to be inserted into the machine again. After all, it is impossible to insert the test card into the machine together with the main body.

In some embodiments, the main body is provided with a plurality of chambers for receiving the testing units. The chambers are configured to fix the testing units. Each testing unit has two positions in the chamber. When in a first position, the testing unit is separable from the chamber. When entering the chamber again, the testing unit is locked by a locking structure previously arranged in the chamber such that the testing unit is inseparable. After the testing unit leaves the chamber and is inserted into the chamber again, it cannot leave the chamber again.

In some embodiments, the testing units are connected with the main body through an easily breakable structure. In some embodiments, the testing unit includes a casing. The casing includes an upper card and a lower card. A testing element is arranged between the upper card and the lower card. The testing element can detect analytes in a liquid sample. One end of the lower card has a first connecting columns and a second connecting columns. The two connecting columns are combined with the main body through an easily breakable or tearable structure, which can be broken when the casing needs to be separated from the main body so as to allow the casing to leave the main body. In some embodiments, the main body also includes two protruding elements, such as plastic columns. The two plastic columns are respectively connected to the first insertion element and the second insertion element through an easily breakable structure, which can be directly broken when the casing needs to be separated from the main body, so as to allow the casing with the remaining first insertion element and second insertion element to leave. The purpose of leaving is insertion into a test result reading machine to complete testing and digital reading of the test results. After the testing is completed, the casing is placed back into the main body. The casing that has been used for testing cannot leave the main body, but is combined with the main body. Specifically, a part of the casing at the end with the insertion elements is inserted into one chamber of the main body and locked by the locking structure in the chamber.

In some embodiments, when the testing unit is inserted into the reading device, the insertion elements are also inserted into the test result reading device. The first insertion element is configured to start a reading program of the test result reading device, and the second insertion element is provided with a label that can be read, which is a label indicating the type of the analyte. In this way, during reading of the test device, it is known that the test result read is the test result representing the type of the analyte or the name of the specific analyte. If the insertion elements do not reach preset positions or the label is not sensed, the reading program will not be started. Actually, the first insertion element and the second insertion element act as a mutual confirmation process. If only one insertion element is identified by the device, the reading program will not be started. Only when the two insertion elements are identified correctly, can the reading program be started. In specific operations, if the reading is started with only one insertion element inserted, although the result is obtained, it is not known which analyte the specific result corresponds to, and an invalid result will be output or the operator will be reminded of an error and to continue the insertion or adjust the insertion position. If the insertion element with the label is identified by the device but the insertion element for starting reading is not identified, the reading will not be started. After the first insertion element is inserted, the reading program can be started. However, if the second insertion element (with the label) is not identified, even if the test result is read, the test result is invalid. Therefore, this can ensure that the test result read is correct, which is different from the traditional process of reading the test result by a machine. Generally, in the traditional process of reading the test result by a machine, the specific test type and the specific test item are fixed in advance, many fixed parameters are preset in the machine in advance, and it is impossible to read other test items by using this machine. For example, a commonly used electronic pregnancy test pen can only test HCG or LH in urine, but cannot test other items. However, the testing elements and the reading device of the present invention have wider applicability. If the test strip is a lateral flow strip, the color appears first. Since the label is a label with granules, the results of a plurality of test items can be read, which, after all, are based on the same principle, i.e., after photos are taken, grayscale values of the photos are analyzed, or after light is emitted, reflected light of the testing area is sensed. That is, results of different items are read by the same process. According to the present invention, items, such as test items, are automatically identified, and different test items can be automatically identified. In addition, these test cards are combined together. The user can freely select the test card according to the test item, and place the test card back to the main body after the completion of the test. After the test card is placed back to the main body, it is inseparable from the main body. In some embodiments, some additional labels may also be arranged. One label represents the test type or item, and the other labels represent the number of insertions.

The reading here includes analyzing the test result of the test result photo taken by the camera and then obtaining a digital result of the test result. Here, the labels may be identified by a physical method such as cooperation between structures, or by sensing with a sensor. For example, in a case that the first insertion element is provided with patterns, numbers or grooves with different widths and the sensor can sense the patterns, the numbers or the grooves with different widths, after the label is identified, a signal corresponding to the sensed label is read from a database preset in the memory, so that a signal of the type of the specific analyte or the name of the specific analyte is sent out, and then, the digital result is finally output based on the result read by the reading element. Specifically, the sensing principle of the sensor is that after the insertion, the sensor senses different structures so as to identify the label based on the patterns or different widths. Alternatively, light emitted may be used to illuminate the pattern or label, so that the label is identified based on the intensity of the light reflected. Of course, the pattern or the label may be photographed so as to identify the type of the analyte.

### Beneficial effect

The device with the above structure can be used for home self-testing. The device with the above structure is convenient to operate and less prone to errors, and reduces the environmental pollution and harm to the operator. In particular, when a plurality of test items are tested, they will not be repeatedly tested or repeatedly read by the reading device. In addition, the name and type of the specific analyte tested can be automatically identified.

### BRIEF DESCRIPTION OF THE DRAWINGS

FIG. 1 is a schematic structural view of a plurality of testing units detachably connected with a main body according to a specific embodiment of the present invention;
FIG. 2 is a schematic structural view after one testing unit leaves the main body according to a first specific embodiment of the present invention;
FIG. 3 is a schematic structural longitudinal sectional view of a test device according to the first specific embodiment of the present invention;
FIG. 4 is a schematic structural view after one testing unit leaves the main body according to the first specific embodiment of the present invention;
FIG. 5 is a schematic structural exploded view of the testing unit according to the first specific embodiment of the present invention;
FIG. 6 is a schematic structural (front) view of testing units detachably connected with a main body (detachably in an initial state) according to a second specific embodiment of the present invention;
FIG. 7 is a schematic structural back view of the device shown in FIG. 6 according to the second specific embodiment of the present invention;
FIG. 8 is a schematic view showing a process of the testing unit being locked and connected with the main body again after leaving the main body and completing testing according to the second specific embodiment of the present invention;
FIG. 9 is a schematic structural view of all the testing units locked and connected with the main body again according to the second specific embodiment of the present invention;
FIG. 10 is a schematic structural longitudinal sectional view after locking and connection again in the second specific embodiment of the present invention shown in FIG. 9;
FIG. 11 is a schematic three-dimensional structural view of the testing unit (containing an insertion end) according to the second specific embodiment of the present invention;
FIG. 12 is a schematic three-dimensional structural view of the testing unit containing two connecting columns according to the second specific embodiment of the present invention;
FIG. 13 is a schematic three-dimensional structural view showing the two connecting columns on the testing unit each provided with color labels corresponding to a type of an analyte and a name of the specific analyte and how they are identified by a sensor according to the second specific embodiment of the present invention;
FIG. 14 is a schematic structural view of testing units connected with a main body (detachably in an initial state) according to a third specific embodiment of the present invention;
FIG. 15 is a schematic structural view after the testing unit leaves the main body according to the third specific embodiment of the present invention;
FIG. 16 is a schematic three-dimensional structural view of the testing unit according to the third specific embodiment of the present invention;
FIG. 17 is a schematic structural exploded view of the testing unit according to the third specific embodiment of the present invention;
FIG. 18 is a schematic structural enlarged view of an insertion end of the testing unit according to the third specific embodiment of the present invention;
FIG. 19 is a schematic structural view of the insertion end of the testing unit locked with a locking structure on the main body according to the third specific embodiment of the present invention;
FIG. 20 shows a schematic structural view of the insertion end of the testing unit locked with the locking structure on the main body, and a schematic structural view of color labels on the locking structure indicating the type of the analyte and how they are identified by the sensor in the device, according to the third specific embodiment of the present invention;
FIG. 21 is a schematic structural view of a testing unit inserted into a reading device according to a specific embodiment of the present invention;
FIG. 22 shows a schematic structural view of the reading device and a schematic structural view showing the position of the sensor for sensing colors on the reading device according to a specific embodiment of the present invention;
FIG. 23 is a schematic flowchart of a sensor sensing different colors and outputting the name of the analyte according to a specific embodiment of the present invention;
FIG. 24 is a schematic structural view of a test result reading device according to a specific embodiment of the present invention;
FIG. 25 is a schematic structural view of a pushing element of a sending device for accommodating a testing unit according to another specific embodiment of the present invention;
FIG. 26 is a schematic structural exploded view of a test result reading device according to another specific embodiment of the present invention; and
FIG. 27 is a schematic structural exploded view of a test result reading device according to another specific embodiment of the present invention.

### DETAILED DESCRIPTION OF THE INVENTION

The structures involved in the present invention or the technical terms used are further explained below. Unless otherwise specified, they shall be understood and explained according to the general terms commonly used in the art.

### Testing

Testing means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, testing means testing an amount of a substance or material. Further, assay also means immunoassay, chemical assay, enzyme assay, and the like.

### Liquid samples

Samples or specimens that can be detected by the test device or collected by the collector of the present invention include biological fluids (such as case fluids or clinical samples). Liquid samples or fluid samples may be derived from solid or semi-solid samples, including feces, biological tissues and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine; and preferably, the biological sample is saliva, sputum, nasal secretion, etc. Food samples include food processing substances, final products, meat, cheese, wine, milk, and drinking water. Plant samples include samples derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water.

An appropriate detecting element or testing element according to the present invention can be used to detect any analyte. These testing elements are all arranged in casings. Preferably, the device of the present invention is used to detect small drug molecules in saliva and urine. Preferably, the device of the present invention may be used to detect small molecular substances such as viruses and bacteria in saliva, or throat or nasal fluid.

### Downstream and upstream

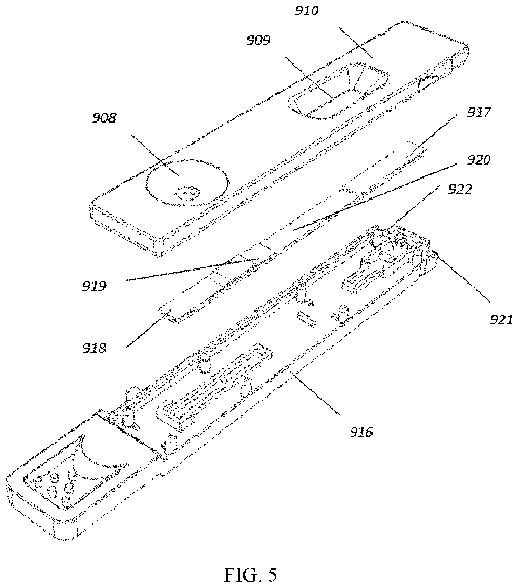

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, as shown in FIG. 5, the testing element 10 provided in the present invention has a sample application area 918, a label area 919, a testing area 920 and an absorption area 917. The sample application area 918 is located upstream of the label area 919. The testing area 920 is located downstream of the label area. The absorption area is located downstream of the testing area. Generally, the fluid flows along the testing element from upstream to downstream. In a specific embodiment of the present invention, when the test device is vertical, for example, as shown in FIG. 5, once the fluid sample contacts the sample application area 918, the liquid sample overcomes the gravity under the capillary action so as to flow upward, i.e., from upward to downward. Thus, the liquid sample flows through the label area 919 to the testing area 920, and finally flows to the water absorption area 917. Then, the liquid sample is added dropwise to a sample application hole 908 on the testing unit and flows to the sample application area 918. Of course, the upstream and downstream here may also be the trajectory or direction of motion of the object, and not the direction of liquid flow.

### Gas communication or liquid communication

Gas communication or liquid communication means that liquid or gas can flow from one place to another. In the flow process, the liquid or gas may pass through some physical structures that play a guiding role. The "passing through some physical structures" here means that liquid passes through the surface of these physical structures or their internal space and flows to another place passively or actively, where passivity is usually caused by external forces, such as flow under the capillary action and the action of air pressure. The flow here may also be a flow due to self-action (gravity or pressure) of the liquid or gas, and also may be a passive flow. The fluid under the action of air pressure may be a forward flow, or also a reverse flow; or a fluid is urged to flow to another position from a position under the action of air pressure. Here, the flow does not mean that a liquid or a gas is necessarily present, but indicates a relationship or state between two objects under some circumstances. In case of presence of liquid, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. Alternatively, two objects are separated at the beginning, combined to achieve a specified function if necessary, then separated, or later combined again for a purpose. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

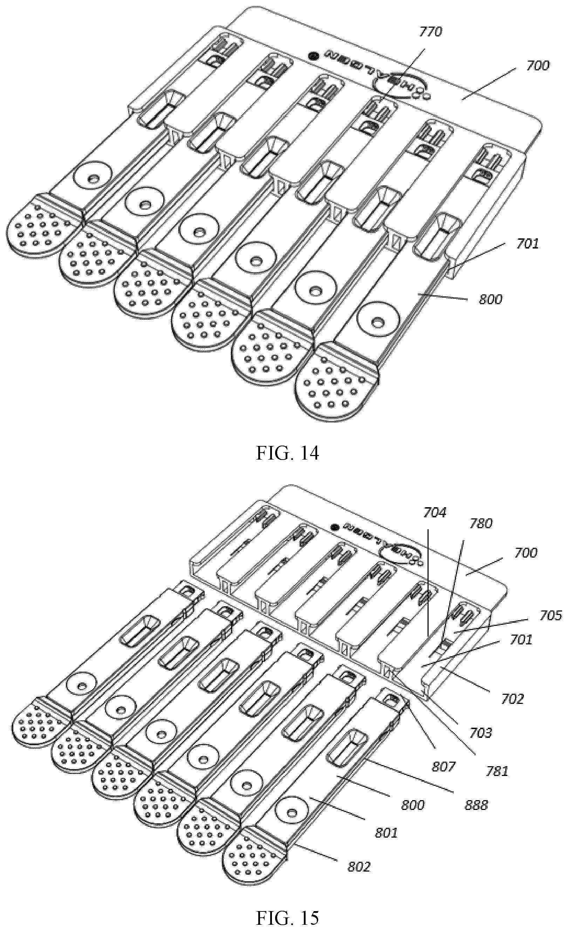

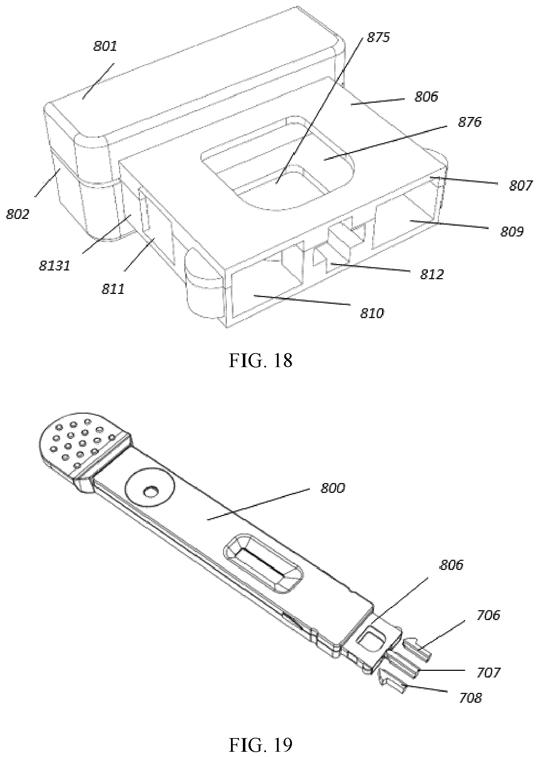

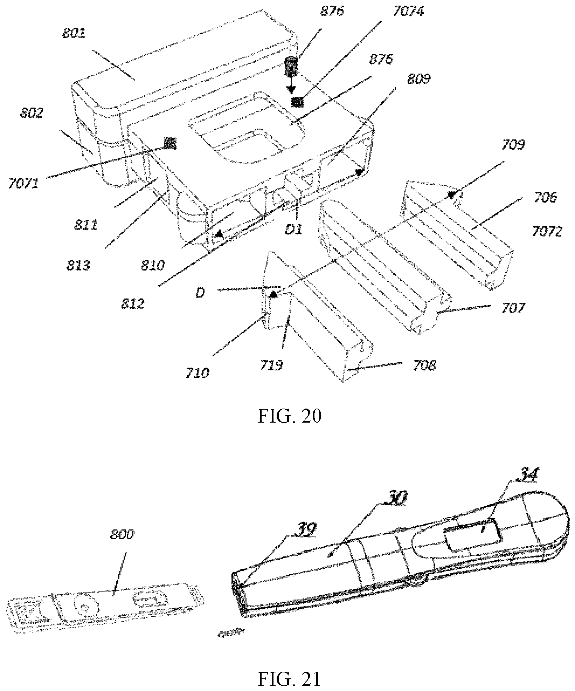

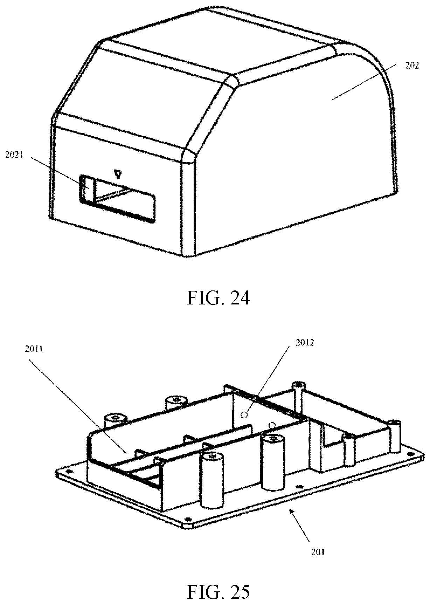

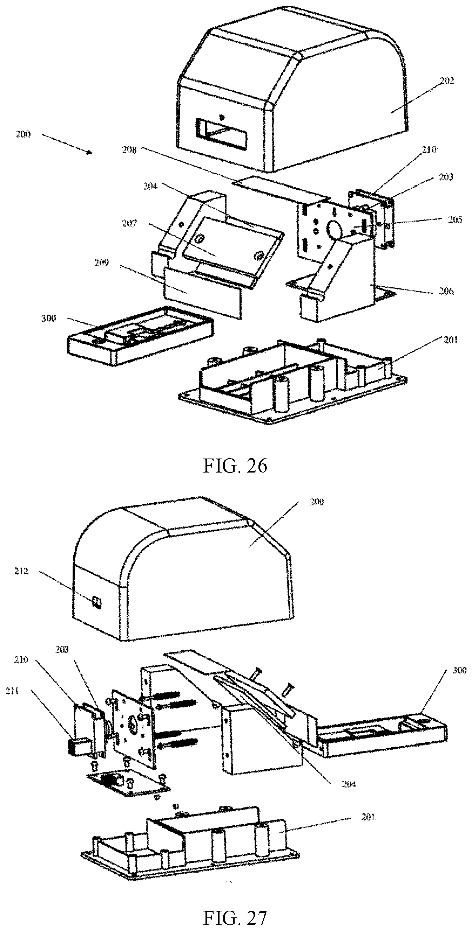

In some embodiments, the components of this detachable combination are combined together in the initial state, and may be separated from each other when needed. When the components are combined again, they cannot be separated from each other, which can avoid confusion or errors. In some embodiments, for example, as shown in FIG. 14 to FIG. 20, the testing unit 800 is combined with the main body 700 in the initial state (FIG. 14). Specifically, the testing unit has an insertion end 806 close to a test window 804, which is configured to be inserted into an insertion chamber 701 of the main body 700. A bottom of the chamber has three locking elements 706, 707, 708. These three locking elements are configured to be inserted into insertion holes at the insertion end of the testing unit so as to allow the testing unit to be in a locked state. This insertion needs the application of a certain force. When the testing unit is in the initial position, as shown in FIG. 14, the testing unit is prevented from leaving the chamber 701 by means of the length of the chamber 701 rather than the insertion of the locking elements into the insertion holes at an end portion 807 of the testing unit 800. More specifically, the chamber has a pair of sliding grooves 703, 704, each including a sliding surface 781 and a sliding closure 702 to form a structure similar to a slide rail. A width between this pair of sliding grooves may be slightly smaller than a lateral width of the testing unit. In this way, when the testing unit 800 is inserted into the chamber 701, the testing unit can be allowed to stay in the chamber 701 without leaving the main body by means of friction between the slide rail formed by the sliding surface 781 and the sliding closure 702 of the sliding groove and a transverse plane 888 of the testing unit (thickness of the upper card and the lower card of the testing unit), and moreover, the lower end portion 807 of the insertion end at the bottom of the testing unit is located on a top end of the locking element (the testing unit is light in weight (generally 50 to 100 g), and when it is naturally placed in the sliding grooves, the locking elements will not be inserted into the holes at the end portion 807 of the insertion end). Generally, the sliding grooves and the testing unit are all plastic elements which can produce friction with each other. In some embodiments, a rough member 780 is arranged on a side wall 705 of the chamber. The rough member 780 is located on the side wall 705 of the chamber and is slightly higher than the side wall 705. In this way, when the testing unit is inserted into the chamber, the testing unit 800 can be fixed into the chamber 701 without easily leaving the chamber 701 by means of the lateral pressure applied by the rough member 780 to the back of the testing unit (the back side of the lower card 802), and the locking elements cannot be inserted into the holes of the insertion end easily. Thus, when testing needs to be performed, the testing unit 800 is directly taken out of the chamber 701, the liquid is added dropwise into the sample application hole 803, such that the liquid flows along the sample application area 918 to the downstream testing area 920, which generally has a T line and a C line. Then, the testing unit is inserted into the reading device to read the test result, mainly optically read the T line area, and output a digital test result (as shown in FIG. 15 and FIG. 21). For example, the testing unit is inserted into a reading device shown in FIG. 21 to FIG. 22 to read the test result (based on the reflection of light), or inserted into a reading device shown in FIG. 24 to FIG. 27 to read the result (in the form of photographing). Of course, the testing unit may be inserted into these reading devices before the liquid is added dropwise into the sample application hole 803, such that the reading device can directly read the test result. After the reading is completed, the testing unit is taken out of the reading device. Generally, the insertion end 806 of the testing unit is inserted into the reading device, and the sample application hole 803 is exposed outside the machine such that the sample can be applied. The testing area is inserted into the device, and irradiated or photographed by an optical element of the reading device, and then the digital result is obtained through analysis. For the specific reading method and the structure of the reading device, details will be described later, and how to read is also not the focus of the present invention.

After the result of the testing unit 800 is read, it is required to take the testing unit out of the device. If the testing unit is discarded at will, or if a plurality of testing units whose test results have been read are placed on a test desk in a case that many test items need to be tested at one time, the testing unit may be easily inserted into the reading device again for reading a second time, such that one testing unit is read two or more times. However, it is difficult for the reading device to tell which test result is correct, causing confusion or error of the results. These testing elements are all single-use products, and the test results of the testing units are valid only within a limited period of time, for example, within 5 to 10 minutes, and the test results read at a time less than or more than this time are invalid. However, if the testing units that have been read are inserted into the device, the device itself cannot determine whether the result is read a second time within the valid time. Thus, among the test results read repeatedly, some results are invalid, which causing confusion to the test result, so that an incorrect result may be output.

As a result, it is expected in the present invention that the testing unit whose result has been read needs to be placed back into the chamber 701 of the main body, which only requires the insertion end 801 at the bottom of the test card to be inserted into the bottom of the chamber. If the locking elements 706, 707, 708 are invisible, this indicates that the testing unit has been inserted into the bottom. Moreover, the testing unit has been locked by the locking elements, and it is impossible to allow the testing unit that has completed first testing to leave the main body 700, which prevents the testing unit from be repeatedly inserted into the reading device. Thus, the testing unit is allowed to be inserted into the reading device only once, and the result is valid only when it is read within the valid time. The "first testing" here includes the process of allowing the liquid to flow for the first time and reading the result of the testing area by the reading device for the first time within the valid time, so the first testing also includes the process of completing the reading of the test result.

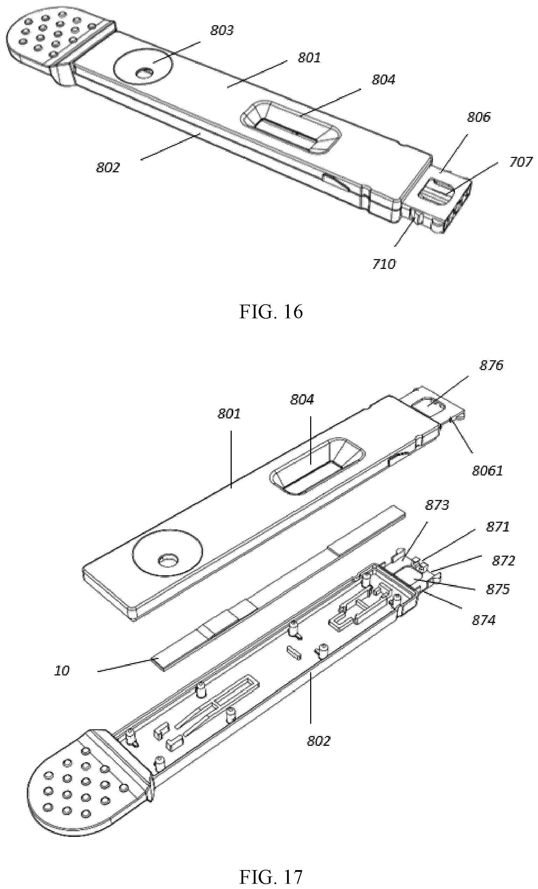

In some embodiments, an insertion end 806 extends from a tail end of the testing unit. The insertion end is provided with insertion holes into which the locking elements at the bottom of the chamber 701 are allowed to be inserted. After the insertion end is inserted, barbs 710 provided at tail ends of the locking elements may be fitted with notches 811 on a side wall of the insertion hole, so that the insertion end is locked. The locking of the insertion end means that the testing unit is locked in the chamber and is inseparable from the chamber 701. In some embodiments, the testing unit includes an upper card 801 and a lower card 802 that are combined together, and a test strip 10 is located between the upper card and the lower card (FIG. 17). The insertion end 806 of the testing unit 800 also includes an upper part and a lower part that are combined together. The upper card is provided with a window 804 that allows the reading device to read the test result. A tail end of the upper card 801 extends out to serve as the upper card 8061 of the insertion end 806, and a tail end of the lower card 802 also extends out to serve as the lower card 8131 of the insertion end 806. The insertion end 806 includes the upper card 8061 and the lower card 813 that are combined together, and the upper card and the lower card are combined together through a hole provided in the upper card and a pin arranged on the lower card. In the combined state, the insertion end 8131 of the lower card is provided with two notches 873, 872 that are covered by the upper card 8061 of the insertion end, such that the insertion holes 809, 810 are formed, and the locking elements 706 and 708 are respectively inserted into the insertion holes. In order to make the insertion end 806 not easily separated after the locking elements are inserted, the locking elements are each provided with a barb 710, 709 which protrudes outward. Side surfaces of the two insertion holes are respectively provided with an outlet 811. At the time of insertion (the maximum lateral distance D between the barbs of the locking elements is generally greater than the maximum distance D 1 between the two insertion holes), the two locking elements move close to each other inward under the action of the insertion holes. After the barbs enter the holes and reach the outlets 811, the barbs naturally move outward so as to extend out of the outlets 811 and be clamped by the outlets 811. Since the outlets each have an outlet edge 813, a surface 719 of the barb is clamped by the edge 713, such that the insertion end is locked, i.e., the insertion end also locks the locking elements, thereby completing the locking of the testing unit. Since the insertion end is assembled together with the upper card and the lower card of the testing unit, the movement of the testing unit is limited, so that the testing unit is locked with the main body 700 without separation.

In some embodiments, the insertion end 806 further has a window 876, and an insertion element 707 is further arranged between the locking elements 706 and 708. The insertion element 707 is inserted into the insertion end, and can be observed through the window 876. On the one hand, when the testing unit is inserted into the chamber 701, this insertion element 707 inserted into the insertion end can be observed through this window, which indicates a correction insertion. On the other hand, the surface of the insertion element 707 is provided with labels, such as patterns or different colors, and these labels can be sensed by the sensor 876 arranged at the reading device, so that the name of the specific analyte corresponding to the test result can be determined, which will be described in detail later. For example, the surface of the insertion element 707 is coated with a green 7074 color area, a purple 7073 color area or a black 7072 color area, and when the insertion element 707 is inserted into the insertion end, the color area can be observed through the window 876, which indicates that the testing unit has inserted into the bottom of the chamber 701 and is in a locked state.

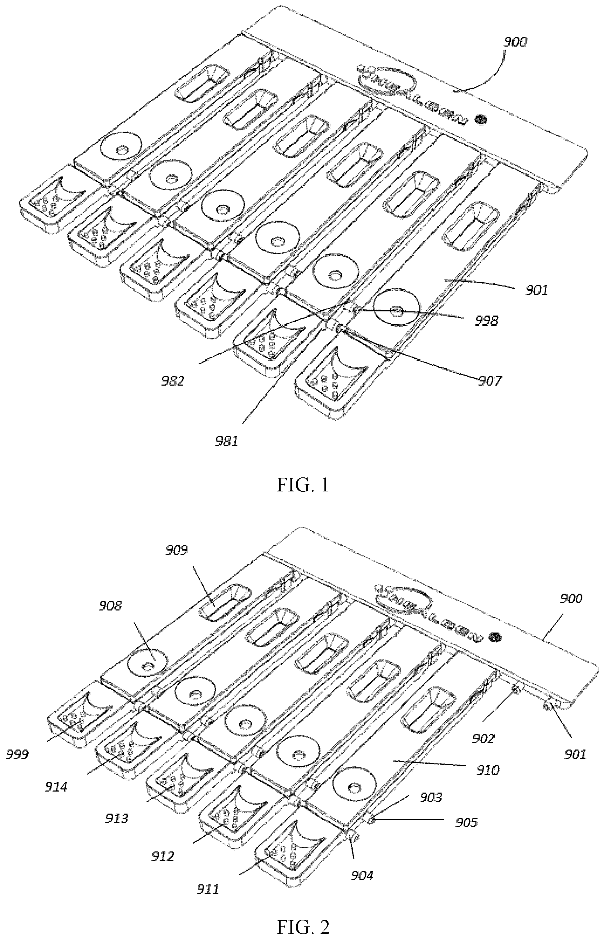

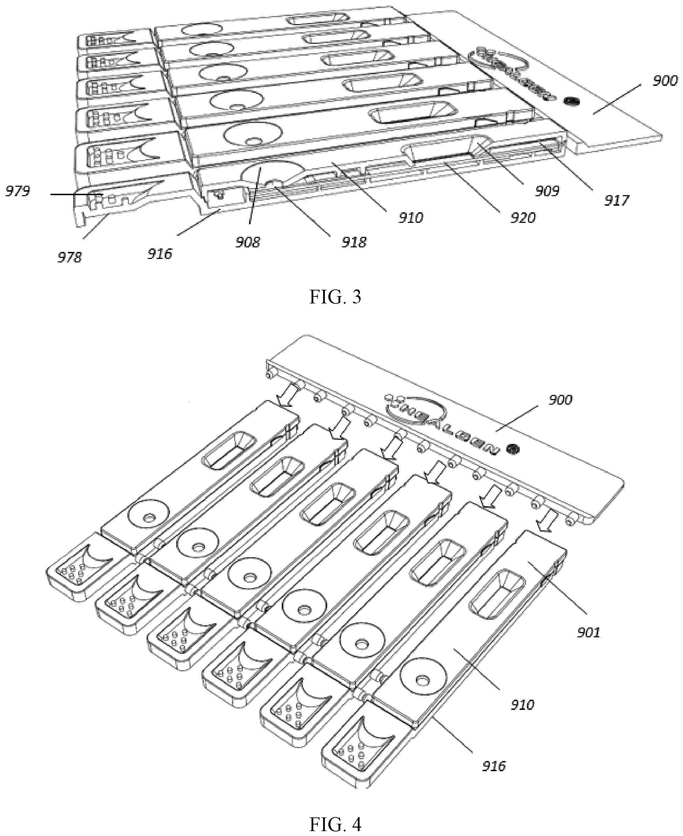

In other embodiments, for example, in another embodiment shown in FIG. 1 to FIG. 5, as shown in FIG. 1, the device has a main body 900. The main body is provided with connecting columns 901, 902, which are respectively connected with connecting columns 922, 921 on the testing unit in an easily breakable manner. According to the easily breakable manner, the position where the two columns are connected may be provided with a plurality of holes, so that end surfaces of the connecting columns are connected, but easily broken, and thus the test card can be separated from the main body. Alternatively, the connecting column 901 may be connected with the connecting column 921 on the testing unit through a very thin connecting column 905. This very thin connecting column has a diameter smaller than or much smaller than the connecting columns 901, 921. In this way, when the testing unit 911 needs to leave the main body 900, this very thin connecting column can be broken. For example, the main body 900 may be repeatedly provided with the same structure, such that the plurality of testing units can be combined together. For example, as shown in FIG. 2, the main body is connected with the plurality of testing units through the connecting columns. For example, the main body 900 is connected with 5 testing units 911, 912, 913, 914, 999, and these testing units may be used for different items or the same item. The test results of these testing units are all read by a reading device, for example an optical reading device, rather than by naked eyes. For example, a reading unit of the reading device is arranged above the test window 909, and light may be allowed to irradiate the testing area 920 of the testing element. In some embodiments, the connecting columns 921, 922 on the test card that are connected with the main body are located at one end of the lower card 916, and the upper card is provided with a sample application hole 908 and a window 909 for displaying the testing area 920. The test result in the window is read not by naked eyes, but by allowing the optical element of the reading device to photograph the testing area 920 or allowing a light-emitting element to irradiate the testing area. For example, the testing area may be photographed, or the light is emitted by an LED and received by a PD, such that the test result is read. During specific production, a plurality of lower cards 916 connected with the main body 900 may be injection-molded at one time, but the upper cards are injection-molded separately. The test strips 10 are placed in clamping grooves of the lower cards, and then the lower cards are covered with the upper cards 910 to form the test device with the plurality of testing units combined, as shown in FIG. 2. Such combined test cards are generally grouped according to according to the major categories of tests, such as infectious diseases, tumor markers, drug of abuse or other major items. In some cases, when the infectious diseases need to be tested, it is required to test a plurality of analytes in the same sample, so that the test needs to be performed many times and the test results need to be read many times. In the embodiment shown in FIG. 1 to FIG. 5, the testing unit is separated from the main body 900 through an easily breakable structure between the connecting columns. Of course, the testing units may also be connected with each other through easily breakable structures. For example, as shown in FIG. 1, the side surface of the lower card 916 of the test card 916 is provided with one or two connecting columns 907, 908, and the lower card of the adjacent testing unit 911 is also provided with corresponding connecting columns 981, 982, such that the testing units are connected through the easily breakable structure. Of course, this solution may be defaulted. When testing needs to be performed, the connecting columns of one testing unit are broken from the main body, so that this testing unit is separated from the main body to perform testing and reading of the test result. If the testing units are directly discarded after use, it will cause confusion. For example, if the testing units that have been used are placed on a test desk, they may be inserted into the reading device again for reading (for example, when fluorescent labels are used), making it difficult to distinguish the testing units that have been used for testing from those that have not been used. When a plurality of test indicators need to be tested at one time, it is prone to confusion. Especially in remote mountainous areas where medical conditions are poor and operators are not professional, it is often easy to make mistakes. For example, if COVID-19 needs to be tested, then the test card 910 should be used. However, if the operator inserts the testing element 910 whose result has been read into the reading device again for reading a second time after the completion of the testing because of panic, an incorrect result will be obtained (the result is not read within the specific time). In some embodiments, the testing unit is provided with a hand-held position having rough protrusions 979, which is convenient to hold. In some embodiments, the hand-held position is not in the same plane as the testing unit, so that the operator can hold and insert the testing unit into the reading device.

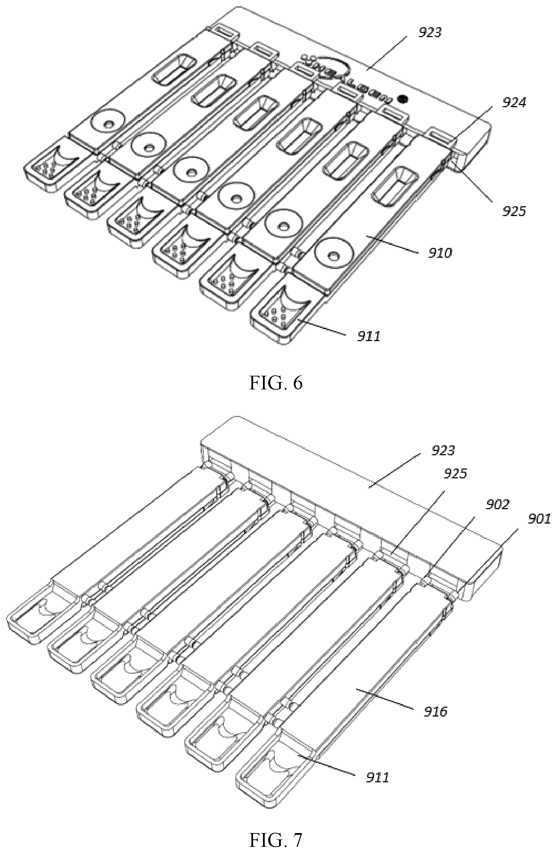

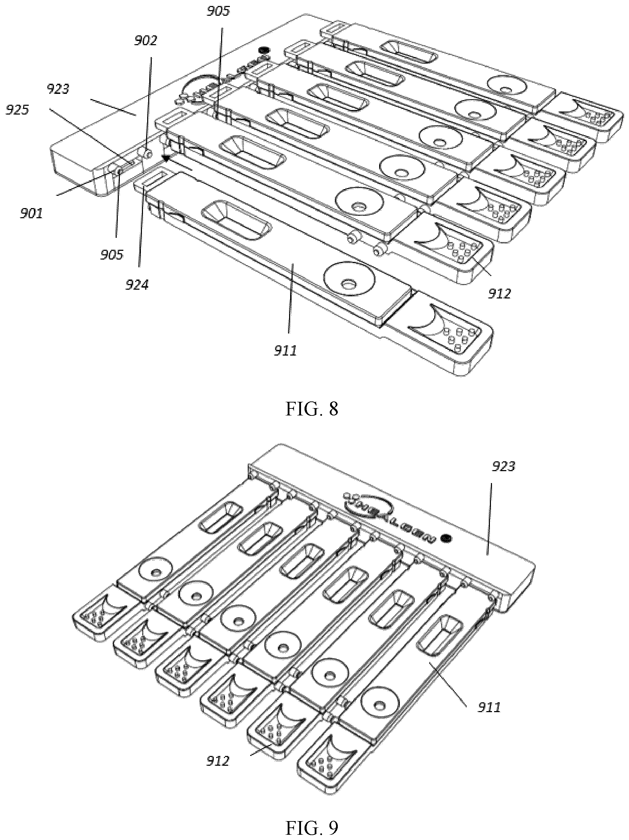

In order to avoid reading a second time or testing a second time, in the present invention, the testing unit that has completed first testing is placed back to the main body 900 and is locked and connected with the main body, so that this problem can be solved. An improved embodiment shown in FIG. 6 to FIG. 13 can overcome the defects in the structure shown in FIG. 1 to FIG. 6. For example, as shown in FIG. 6 to FIG. 7, the tail end of the testing unit is provided with two connecting columns 921, 922, which are respectively connected with the connecting columns 901, 902 on the main body 923 in an easily breakable manner. In some embodiments, an insertion end 924 extends from the tail end of the upper card 916 of the test card. In an initial state, the extending insertion end 924 covers the surface of the main body 732, and the tail end of the lower card 910 of the same testing unit is connected with the connecting columns 901, 902 on the main body through the connecting columns 921, 922 through an easily breakable structure (FIG. 6 shows the front side, and FIG. 7 shows the back side). The insertion end 924 is provided with an opening 9251. The main body 923 is provided with an insertion hole 925 near the connecting column, and a locking element 926 is arranged in the hole or chamber 925. The locking element has a hook 927. During specific operations, with the breakable structure, when the testing unit is placed back to the main body after leaving the main body 923 and completing testing (including the first testing and first automatic reading of the test result), the insertion end 924 is inserted into the hole 925, and the hook 927 of the locking element 926 arranged in the hole is passed through the opening 9251 of the insertion end, so that the testing unit is locked in the hole 925. With this structural design, the testing unit is inseparable from the main body 923, thereby completing fixation (FIG. 8 is a schematic view of the insertion process, and in FIG. 9, all of the 5 testing units are locked to the main body 923 without separation in the same manner).

### Testing element

The "testing element" used herein refers to an element that can be used to detect whether a sample or a specimen contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the present invention.

Various testing elements can be combined for use in the present invention. One form of the testing element is a test strip or a lateral flow test strip. The test strips used for analyzing the analyte (such as drugs or metabolites that show physical conditions) in samples can be of various forms such as immunoassay or chemical analysis. The test strips may adopt a non-competitive or competitive analysis mode. A test strip generally contains a water absorbent material that has a sample application area, a reagent area, and a testing area. Fluid or liquid samples are added to the sample application area and flow to the reagent area under the capillary action. If analyte exists in the reagent area, samples will bind to the reagent. Then, the samples continue to flow to the testing area. Other reagents such as molecules that specifically bind to the analyte are immobilized in the testing area. These reagents react with the analyte (if any) in the sample and bind to the analyte in this area, or bind to a reagent in the reagent area. The label used to display the detection signal exists in the reagent area or the detached label area.

In a typical non-competitive analysis mode, if a sample contains the analyte, a signal will be generated; and if not, no signal will be generated. In a competitive method, if no analyte exists in the sample, a signal will be generated; and if the analyte exists, no signal will be generated.

The testing element may be a test strip, which may be made of a water absorbent material or non-water absorbent material. The test strip may contain several materials used for delivery of liquid samples. One material of the test strip can cover the other material thereof. For example, the filter paper covers the nitrocellulose membrane. One or more materials may be used in one area of the test strip, and one or more other different materials may be used in the other area. The test strip can be stuck to a certain support or on a hard surface for improving the strength of holding the test strip.

The analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal may be in the sample application area, the reagent area or the testing area, or on the entire test strip, and one or more materials of the test strip may be filled with this substance. The solution containing a signifier is added onto the surface of the test strip, or one or more materials of the test strip is or are immersed in a signifier-containing solution. The test strip containing the signifier solution is dried.

Various areas of the test strip may be arranged in the following way: a sample application area, a reagent area, a testing area, a control area, an area to determine whether the sample is adulterated or not, and a liquid sample absorption area. The control area is located behind the testing area. All areas can be arranged on one test strip that is only made of one material. Alternatively, different areas may be made of different materials. Each area can be in direct contact with the liquid sample, or different areas are arranged according to the flow direction of liquid sample; and a tail end of each area is connected and overlapped with the front end of another area. Materials used can be those with good water absorption such as filter paper, glass fibers or nitrocellulose membranes. The test strip may also be in other forms.

The nitrocellulose membrane test strip is commonly used, that is, the testing area includes a nitrocellulose membrane (NC) on which a specific binding molecule is immobilized to display the test result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or test device of the present invention for the test of an analyte, for example, the test of an analyte in a sample.

Test strips used in the present invention may be commonly referred as lateral flow test strips. The specific structure and testing principle of the test strips are well known to a person skilled in the art in the prior art. A common test strip 10 (FIG. 5) includes a sample collection area or a sample application area 918, a label area 919, a testing area 920 and a water absorption area 917; the sample collection area includes a sample receiving pad, the label area includes a label pad, and the water absorption area may include a water absorbent pad, where the testing area includes necessary chemical substances for detecting the presence or absence of analyte, such as immunoreagents or enzyme chemical reagents. The nitrocellulose membrane test strip is commonly used, that is, the testing area 920 includes a nitrocellulose membrane, and an area (for example, a T-line in the form of lines) on which a specific binding molecule is immobilized to display the test result; and other test strips such as cellulose acetate membrane or nylon membrane test strips can also be used. Of course, in the downstream of the testing area, there may also be a test result control area (C-line); and generally, the control area and the testing area appear in the form of horizontal lines, namely, a test line or a control line. Such test strips are conventional. Of course, they may also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the test device of the present invention or can be disposed in contact with the liquid samples in a testing chamber or used to detect the presence or absence of an analyte in the liquid samples that enter a testing chamber, or the quantity thereof.

### Testing unit and label of test type

The testing unit here refers to the casing containing the testing element 10, which can be used for testing individuals of analytes in a sample. Generally, the testing unit here is the casing containing the testing element. The casing is generally formed by combining an upper plastic piece and a lower plastic piece. The testing element is arranged between the two plastic pieces. The upper plastic piece includes a sample application hole and a test result reading window. The sample application hole corresponds to the sample application area of the testing element, and the test result reading window corresponds to the testing area of the testing element. When the testing unit is inserted into the machine, the test result reading unit in the machine is aligned with the testing area of the testing element to read the test data, and then outputs the digital test result. In addition, the testing unit is further provided with two limiting structures. When the testing unit is inserted into the reading device to read the test result, the limiting structures are inserted into a reading end of the device in fixed positions, so that the reading element (photographing or optical element) can correspond to the testing area and the test result of the testing area can be read.

When a machine is used to read the test result of the testing unit, the testing unit generally corresponds to one test item or one analyte in one sample. However, in some cases, different testees may be subjected to one or more test items. In this case, it is expected to collect the sample only once. If a plurality of testing units are fixed or combined together, one testing element corresponding to the desired test item may be taken out of a combination of testing units and then inserted into the machine to read the data. On the contrary, if the plurality of test cards are not combined together, when a plurality of items need to be tested, it is required to take out different test items from different packing boxes, which is cumbersome and error-prone. In the present invention, different test items are combined together, so that when a plurality of items need to be tested, it is required to collect only one sample, add the sample dropwise respectively to the plurality of testing units and then take the individual testing units from the combination to be read by the machine. In some embodiments, after the testing is completed, the plurality of testing units that have been used are combined together. Once these testing units are combined, they are unable to be used for testing again, which can prevent misoperation. The testing units that are combined again are inseparable, which has been specifically described above.

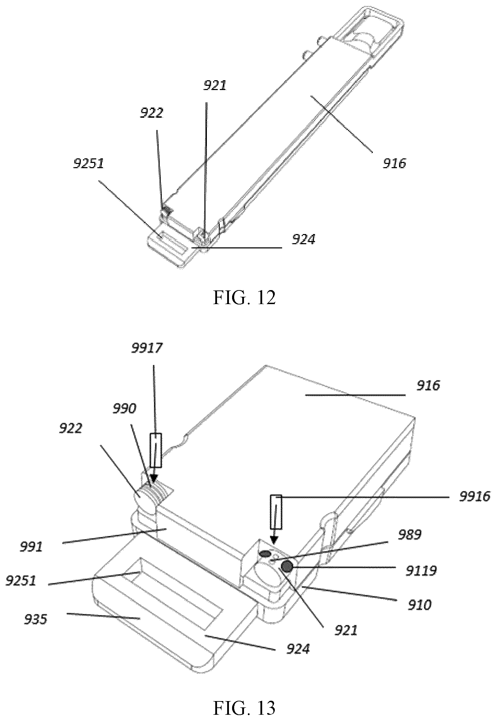

For example, as shown in FIG. 2, the main body has 5 testing units 910, 911, 912, 913, 914 that are combined together and located on one main body 900. All these 5 units can be used for testing viruses or drugs or tumors in the sample, but these testing units correspond to different specific markers or objects. For example, all the 5 testing units are used for testing viruses, including 5 viruses, for example, influenza A virus, influenza B virus, COVID-19, hepatitis B virus or hepatitis A virus. Since each testing unit corresponds to a different analyte, it is necessary for the reading device to automatically identify the specific analyte represented by the reading result. A traditional method may include spraying a QR code on the testing unit and then scanning the QR code. However, in the case that the test result on the testing area 920 is read by emitting light by an LED and receiving light by a PD, the QR code or bar code cannot be identified. If the reading device is equipped with an element capable of scanning QR codes or bar codes, the reading device will be huge and expensive, which is not conducive to home testing. In order to overcome this problem, the testing units of the present invention are provided with some labels. These different labels can be read by the reading device such that the type of the analyte tested or the name of the specific analyte tested can be identified. In this way, even if the testing units have the same appearance, the machine can easily identify the specific analyte represented by the reading result when reading the test result of the testing area. In some embodiments, as shown in FIG. 12 to FIG. 13, the testing unit is provided with two connecting columns 921, 922. The two connecting columns are connected with the connecting columns 901, 902 on the main body 900 through easily breakable structures, so that the testing unit can be easily separated from the main body. The first connecting column 921 and the connecting column 9922 left on the testing unit are provided with different labels, which can be sensed by sensors 9917, 9916 previously arranged in the reading device to produce different electrical signals. The electrical signal is transmitted to a controller. The controller may enable a computing center to retrieve previously stored data corresponding to the label (data of the type of the analyte or the name of the analyte corresponding to the specific label) so as to identify the data of the test type and the specific analyte. For example, data of the following table may be previously stored in the reading device. Each testing unit has a different label, so that information identified by the reading device is different, thereby effectively distinguishing the test items.

**Table 1: Relationship between test type and specific analyte.**

| | Item | Item | Item |
|---|---|---|---|
| Depth of small hole 989 in first column 921 | 1 mm/virus | 2 mm/DOA | 1.5 mm/tumor marker |
| Color 990 on second column 922 | Red/COVID-19; yellow/influenza A, black/influenza B; purple/hepatitis B virus, green/hepatitis A virus. | Red/morphine; yellow/THC, black/heroin. | Red/hemoglobin; yellow/CEA, black/CA125. |

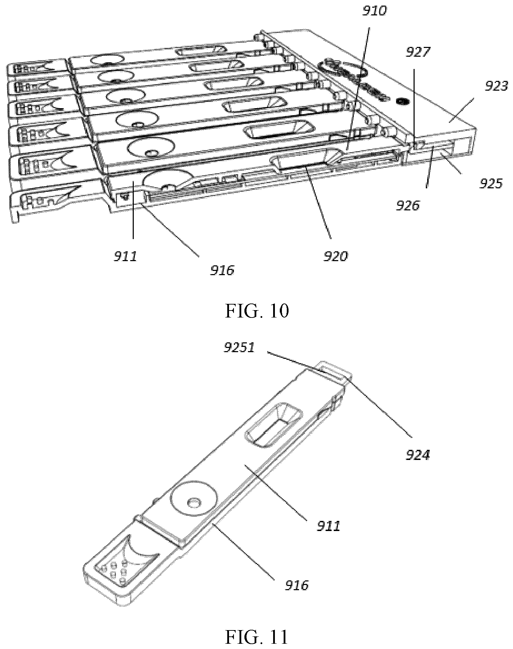

The above data is previously stored in a memory of the reading device. The data can be retrieved by the reading device. For example, the sensor senses the color to produce an electrical signal, the electrical signal is sent to the controller, the controller sends out a signal to enable an analyzing or computing center to investigate the stored information and transmits the retrieved information to an output module, and the test type or the name of the specific analyte is displayed on a display. The test result on the testing area 920, such as positive or negative, or the specific quantity, is also displayed on the display. For example, in a case that the depth of the small hole 989 in the first column 922 of one test card is 1 mm and the color on the second column is black, when the testing unit is inserted into the reading device, the sensor 9916 for sensing depth and the sensor 9917 for sensing color, which are previously arranged inside the reading device, are disposed on or around the connecting columns and electrically connected with a control module through wires, so that these sensors can be used for transmitting signals. The sensors convert the sensed signals (depth and color) into an electrical signal, which is transmitted to a controller. The controller outputs a signal that enables the computing center to retrieve corresponding information from the memory. For example, if the retrieved information includes a depth of 1 mm which represents an infectious disease and black which represents influenza B, it indicates that the test item of the inserted testing unit is virus, and the specific item or the specific analyte is influenza B. The computing center sends the signal to the output module which includes the display, and "the test item is an infectious disease, and the specific analyte is influenza B" is displayed on the display. At the same time, the test result, for example, positive or negative, or the specific quantity, is also output by an output unit. Thereby, the information output by the output unit includes the test item or the specific analyte and the test result. For example, what is displayed is influenza virus B, and the test result is positive. Such sensors for sensing color or identifying color are commonly used accessories in industry, and can be purchased and mounted inside the reading device. These sensors are located on a PCB together with the electronic device for reading test results and the memory. Of course, the two columns may be both be coated with color labels and combined to indicate the test type or the specific analyte. For example, the first column is coated with black which indicates virus, and the second column is coated with red which indicates influenza A and purple which indicates influenza B. It can be understood that one column is coated with two color areas which are identified by one sensor for sensor color. Black represents virus, and purple represents influenza B. Such manners are all available. It can be understood that the connecting column is coated with only one color label which represents the specific analyte. For example, as shown in FIG. 10, words on the main body 923 indicate that the 5 testing units are used for testing infectious diseases and also indicate the names of the 5 specific viruses, but do not indicate the name of the specific virus corresponding to each test card. When one person needs to be subjected to 5 test items at the same time, the present invention is especially useful. It is only required to add the sample dropwise into the sample application hole 908 and insert the testing unit into the reading device for reading. At this time, reading the testing area by the reading device and identifying different colors on the connecting column by the sensor are carried out at the same time. In this way, after the reading is completed, the test result and the specific test item represented by the identified color are transmitted to the output unit so as to be combined, and the output unit finally outputs the result, which indicates the specific test type and the specific test result. The label here may be, for example, color, pattern or depth of hole, and any label that can be sensed by a sensor is available in the present invention. For convenience, the color label on the connecting column may be a color pattern or color area that is printed by an ink-jet printer or drawn with ink. As for how to make a sensor sense different colors and retrieve the corresponding test type or item from a memory, it is easy to implement by a person of ordinary skill. In this way, the function of automatically identifying test items is achieved.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of abuse of drug include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the present invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the present invention. In some embodiments, the treatment liquid contained in the receiving device does not contain analytes.

As described above, a plurality of analytes may be tested at the same time. During testing, the result of the testing area is automatically read by the reading device, and at the same time, the result is output by an output device, for example, a display or a printer which can directly tell the test result. This is especially suitable for home self-testing or testing for small clinics in remote mountainous areas.

### Reading device

The testing units 800 with different testing elements may be inserted into the test result reading device 30 such that the test result on the testing area 920 can be read. The reading device is provided with an electronic reading element which can read the test result of the testing area 920 of the testing element in the testing unit 800. The reading is generally based on the optical principle, which is similar to the existing electronic early pregnancy reading method. Light is emitted to irradiate the testing area 920, for example, the T line. The light reflected from the testing area (T line area) is received by a photoelectric sensor (PD), and the reflected light received is converted by the photoelectric sensor into an electrical signal. Through computing, a digital result of the concentration of the analyte is output and displayed on a display screen. The methods of reading the test result on the testing area 920 and how to read the test result have been specifically described in CN104730229, CN101650298, US5,58079, US7,315,378 and US application No. 17/576,364 filed by the applicant, and are not the focus of the present invention. Based on this principle, test results of different analytes can be read. In this reading device, the sensor 876 for sensing different colors is provided. The sensor is arranged on a PCB, and an LED and a device for receiving reflected light from the testing area are also arranged on the PCB so as to read the test result on the testing area.

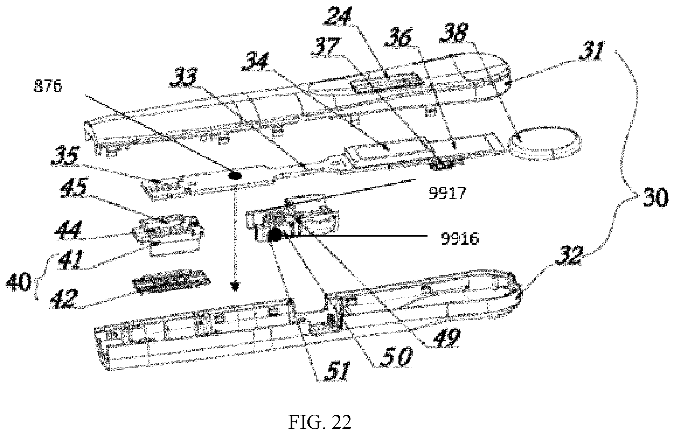

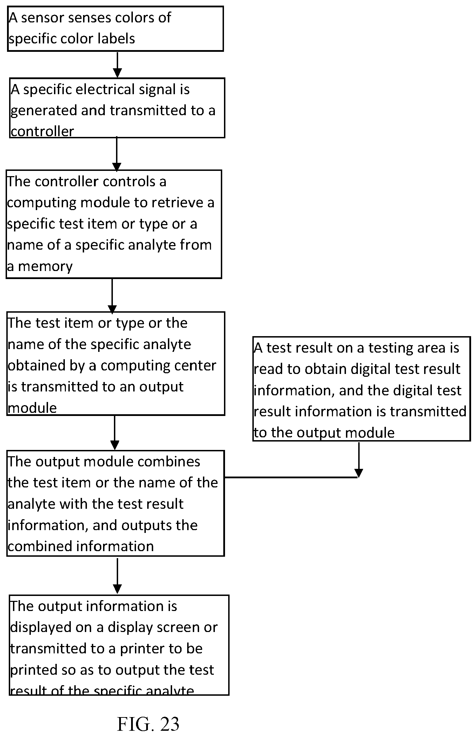

For example, in the reading device shown in FIG. 22, when the insertion end of the testing unit, for example, the testing unit shown in FIG. 11 to FIG. 12, is inserted into the reading device, two elastic clamping pieces 51 in the device clamp clamping grooves of the testing unit, and the insertion end of the testing unit comes into contact with a spring 49 in the device. The first column 921 and the second column 922 on the back side of the insertion end are located either between or below the elastic clamping pieces 51. If the first column and the second column are located between the elastic clamping pieces, then the two elastic clamping pieces are respectively provided with two sensors 9916, 9917. The sensor 9916 corresponds to the connecting column 921 and is used for sensing the depth in the connecting column, and the sensor 9917 corresponds to the connecting column 922 and is used for sensing the color. If the connecting columns are located below the elastic clamping pieces 51, a lower casing 32 of the reading device may be provided with two sensors which respectively correspond to the connecting columns 921, 922. It can be understood that these sensors and the electronic test result reading element on the PCB are controlled by the same controller, and are all electrically connected to the computing center and the output module so as to transmit electronic signals. The reading element 35 on the PCB includes two LEDs for emitting light, and a PD for receiving light in the middle. The light emitted by the LEDs irradiate the testing area 920, the PD receives the reflected light, and the concentration of the analyte is calculated according to the intensity of the reflected light. The output digital result may be displayed on a display screen 34. These systems are powered by a button battery 38. A light guide hole is provided below the LEDs and the PD, so that the light emitted by the LEDs can irradiate the T line area or C line area of the testing area. The PD receives the light reflected from the testing area, and digitally outputs the quantity of the analyte according to the amount or intensity of the reflected light. The specific reading and outputting methods are the conventional prior art, and have been specifically described in CN104730229, CN101650298, US5,58079, US7,315,378 and US application No. 17/576,364 filed by the applicant. In some embodiments, the reading device is further provided with one or two sensors for sensing the labels on the testing units. These labels indicate the specific test types or the names of the analytes. For example, in some embodiments, as shown in FIG. 20, in a case that the surface of the insertion end 806 of the testing unit is coated with a color label, for example, a green label 7071 or a black label 7074, the names of the specific analytes corresponding to the colors may be previously stored in the memory. For example, green corresponds to COVID-19, and black corresponds to influenza B virus. The corresponding relationship may be changed at will, for example, the relationship shown in Table 1 is acceptable. If the analyte corresponding to the black label 7074 is influenza B virus, it indicates that the object in the sample in this testing unit is influenza B virus. In the reading device, the sensor 876 is arranged on the PCB and electrically connected with the test result reading system, and can transmit the sensed signal to the controller and the computing center, and the test result is finally transmitted to the output module, for example, the display. The specific flowchart is shown in FIG. 23. When the testing unit is inserted into the reading device, the position with the black label is allowed to be located below the sensor 876. At this time, the LEDs and the PD for reading the testing area are located on the window 804 through a light guide structure, and the test result on the testing area 920 of the testing element corresponding to the window is read. The sensor 876 senses the black color on the insertion end 806 so as to produce a specific electrical signal and send the electrical signal to the control center. The control center sends the signal to a computing module and retrieves the name of the analyte corresponding to black from the memory. Then, the computing center transmits the name of the specific analyte retrieved from the memory, for example, influenza B, to the output module. At the same time, the digital information obtained by reading the test result on the testing area is also transmitted to the output module. The output module combines the test result and the name of the specific analyte, such that the result is displayed on the display 34. Of course, in the reading device, the PCB is provided with a structure for mounting the battery 38. The PCB has a neck 33, and the spring 49 is arranged below the neck. After the testing element is inserted, the end portion 807 at the insertion end contacts the spring so as to be in a fixed position in the reading device. The display screen 34 is exposed through a window of an upper casing 31, and the result information, including the name of the analyte and the numerical value of the test result, may be observed through the window. In in some embodiments, the reading device further includes a limiting structure, which ensures the testing unit to be correctly inserted into the reading device. Specifically, the limiting structure 40 includes a first limiting member 41 and a second limiting member 42. The first limiting member 41 and the second limiting member 42 are assembled together to form the limiting structure 40. The second limiting member 42 is directly mounted on the first casing 31 or the second casing 32. The first limiting member 41 is mounted on the second limiting member 42. The PCB 33 is mounted on the first limiting member 41.

The color sensor is mainly based on two working principles: photoelectric effect and trichromatic theory. The photoelectric effect is a phenomenon in which when light irradiates the surface of a substance, the substance absorbs light energy and release electrons. The color sensor identifies the color by absorbing light with a specific wavelength based on the photoelectric effect. The trichromatic theory simply means that any color can be mixed from three primary colors (red, green and blue). The color sensor works based on this theory. In the color sensor, three different photoelectric elements are usually used to respectively sense red, green and blue light. When light irradiates these elements, different electrical signals are produced, and the magnitudes of these signals are related to the color components. By measuring the magnitudes of the three signals, the color of the light can be determined. There are three types of color sensors available in the present invention: a filter-type color sensor, an RGB color sensor and an RGB color convertor. The filter-type color sensor uses a filter to remove light with a specific wavelength so as to detect a certain primary color. This sensor is simple in structure and low in price, and is suitable for application scenes where a single color needs to be detected. The RGB color sensor uses three photodiodes to respectively detect the three primary colors (red, green and blue). By measuring output currents of the three diodes, the color of the mixed light can be determined. This sensor has high accuracy, and is suitable for application scenes where multiple colors need to be detected. The RGB color converter is a device that converts color into RGB values. By decomposing the color into a proportion of the three primary colors (red, green and blue), the color is converted into a digital signal, which can be processed and identified by a computer conveniently. This converter is widely used in the fields of computer vision and image processing. Any sensor mentioned above is available for the color sensor of the present invention. Generally, the filter-type color sensor, which is low in price, is used to detect a single color. For example, the sensor 876 is a filter-type color sensor which is used to detect the solid color, for example, black or green.

In this way, when testing needs to be performed, the sample is collected and added dropwise into the sample application hole 803 of the testing unit. Then, the testing unit is inserted into the reading device 30 through an inlet 39 so as to read the test result of the test strip. The carrier is allowed to be in a locked position. After the reading of the test result is completed, the casing is automatically ejected out of the reading device or the testing unit 800 is manually pulled out, and then the testing of the next testing unit is performed. If the testing element contained in the testing unit is used for detecting one analyte, the testing can be easily completed with this device. When there are a plurality of testing units that correspond to different analytes, the reading device needs to be able to identify the specific analyte corresponding to the test result read. Therefore, the testing units of the present invention are provided with some indication labels which can be identified by the reading device. After the indication labels are identified, the specific analyte corresponding to the test result read is sent to the reading device, so that the test result is more accurate.

In other embodiments, when the testing area reading device is in the form of photographing, the line on the testing area 920 is acquired by photographing, and then the signal strength on the test line is analyzed. The specific analysis method is specifically described in Chinese application No. 201210141387.9, which mainly describes how to read the result of the test line on the test strip, take photos, and analyze the test result on the photo, specifically using gray values. All the contents of this patent are included as part of the present invention. FIG. 24 to FIG. 27 show another device for reading the test result on the testing area 920 of the test strip. The device includes an external casing 200, which has an insertion opening 212. The inside of the device includes a photographing element 210, which is similar to a camera. A lens 203 for photographing extends out of a hole in a partition 205, and a mirror 204 is arranged on the opposite side. The mirror is located on a base 300 at an angle of 45 degrees. Testing units may be placed on the base 300. The base is located on a base support 201. The base support 201 may automatically extend and retract through the opening 212 in the casing. When testing needs to be performed, the base support 201 with the base 300 extends out of the casing 200, the testing unit is placed on the base. Once the testing unit is placed on the base, the base enters the device and is located below the mirror 204, so that an image of the entire testing unit is formed on the mirror. The camera takes a photo of the testing unit, especially a photo of the structure on the testing area 920 of the testing unit, through the mirror. Then, the color depth on the testing area in the photo is analyzed so as to determine the quantity of the analyte. The mirror is provided with a plurality of baffles 208, 209, 206, which are used for preventing outside light from irradiating the mirror. The baffle 208 may be provided with a plurality of light-emitting elements for irradiating the base 300, which provides a bright environment for photographing. Such methods for reading the test result are known in the prior art, and for specific descriptions, reference may be made to Chinese application No. 201210141387.3 and No. 201120573872.9. The focus of the present invention is not how to obtain the test result of the testing area by photographing, but the arrangement of sensors in the reading device to sense labels, especially color labels, on the testing unit. The positions of the sensors are also not important, as long as the sensors can sense different colors. Thus, names of different analytes can be identified based on different colors, so that the specific analyte corresponding to the test result can be automatically determined. When the main body contains a plurality of testing units, the name of the analyte tested by each testing unit can be automatically identified, thereby avoiding confusion, which is different from the traditional similar identification methods.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where such restriction is not specifically described here. For example, the terms "comprising", "essentially consisting of" and "consisting of" in each embodiment herein may be replaced by the rest 2 terms. The so-called "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. Any person of ordinary skill in the art can make some modifications and changes according to the description of the present invention. These modifications and changes are also considered to fall within the scope of the present invention and the scope limited by independent claims and dependent claims.

## Claims

1. A device for detecting analytes in a liquid sample, comprising a main body, wherein the main body comprises a plurality of testing units, each testing unit comprising a testing element therein, and the testing element being configured to detect one or more analytes in the liquid sample; the testing units are capable of being detachably combined with the main body; when testing needs to be performed, each testing unit is separable from the main body; and the testing unit is configured to be inserted into a test result reading device for automatic reading of test results.

2. The device according to claim 1, wherein after the testing unit is separated from the main body, the testing unit is capable of being combined with the main body again after the reading of the test result is completed.

3. The device according to claim 3, wherein when the testing unit is combined with the main body again, the testing unit is locked with and inseparable from the main body.

4. The device according to claim 4, wherein the main body comprises a locking element, and the locking element is configured to make the testing unit fixed in the main body without separation after the testing unit leaves the main body, completes testing and returns into the main body.

5. The test device according to claim 4, wherein the main body comprises an insertion chamber, the locking element is elastic and is provided with a barb and located in the insertion chamber, the testing element comprises an insertion end, and the insertion end comprises an insertion hole, wherein after the testing unit completes the reading of the test results, when the insertion end is inserted into the insertion chamber, the locking element is inserted into the insertion hole on the insertion end such that the testing unit is locked to the main body and connected with the main body into an integrated structure.

6. The device according to claim 5, wherein when the testing unit is in an initial state before leaving the main body, the testing unit is not locked, but is located on the locking element, kept in the insertion chamber by means of friction between an insertion slot of the insertion chamber and the testing unit and detachably combined with the main body.

7. The device according to claim 4, wherein the main body has connecting columns, and the testing units also comprise connecting columns; and the connecting columns on the main body are connected to the connecting columns on the testing units in an easily breakable manner.

8. The device according to claim 7, wherein the main body comprises an insertion chamber, each testing unit comprises an insertion end, the insertion end has an opening, and the locking element is located in the insertion chamber and provided with a barb; and after the testing unit completes the reading of the test results, when the insertion end of the testing unit is inserted into the insertion chamber, the barb passes through the opening such that the testing unit is locked with the main body without separation.

9. The device according to claim 8, wherein the testing unit comprises a casing, and the casing contains the testing element; and one end of the casing comprises the connecting columns, and the connecting columns are provided with labels indicating a test item or type or a specific analyte, the labels being automatically identifiable by the reading device.

10. The device according to claim 9, wherein the casing comprises first and second connecting columns, the first connecting column comprising a label indicating a type of the analyte tested, and the second connecting column comprising a label indicating a name of the specific analyte.

11. The device according to claim 10, wherein the labels are in a form of patterns, numbers, grooves with different widths, threads with different widths, or different colors.

12. The device according to claim 11, wherein the device has a sensor or an optical sensor for sensing the labels so as to identify the labels, such that a signal generated after identifying the label is transmitted to a control center and the control center allows a computing center to retrieve the corresponding type of the analyte or the name of the analyte from a storage device of the device.

13. The device according to claim 12, wherein the sensor in the device is a sensor capable of sensing different colors.

14. The device according to claim 13, wherein the types of the analytes comprise drugs, infectious diseases and tumor markers.

15. A system for detecting analytes in a liquid sample, comprising a test device and a device for reading test results of a testing area on the test device, wherein the test device comprises a main body, and the main body comprises a plurality of testing units, each testing unit comprising a testing element, and the testing element being configured to detect one or more analytes in the liquid sample; the testing elements are capable of being detachably combined with the main body; when testing needs to be performed, each testing unit is separable from the main body; the testing unit comprises the testing area; the testing unit is configured to be inserted into the test result reading device for automatic reading of the test results on the testing area; and the testing unit comprises a color label of a name of a specific analyte; and the device comprises a sensor for sensing colors.

16. The system according to claim 18, wherein after sensing the color, the sensor generates an electrical signal and transmits the electrical signal to a control center, the control center retrieves the name of the specific analyte from data of names of analytes corresponding to colors and transmits the name of the specific analyte to a computing center, and in the computing center, output signal data is generated based on the name of the specific analyte and the test result and thus transmitted to an output device to be output.
